(19) Europäisches Patentamt
European Patent Office
Office européen des brevets

(11) Veröffentlichungsnummer: **0 255 801 B1**

# (12) EUROPÄISCHE PATENTSCHRIFT

(45) Veröffentlichungstag der Patentschrift: **28.11.90**

(51) Int. Cl.[5]: **C07D 251/70,** C08K 5/34

(21) Anmeldenummer: **87810425.6**

(22) Anmeldetag: **27.07.87**

(54) **Anilinotriazine und deren Verwendung.**

(30) Priorität: **31.07.86 CH 3083/86**

(43) Veröffentlichungstag der Anmeldung:
**10.02.88 Patentblatt 88/6**

(45) Bekanntmachung des Hinweises auf die Patenterteilung:
**28.11.90 Patentblatt 90/48**

(84) Benannte Vertragsstaaten:
**DE FR GB IT**

(56) Entgegenhaltungen:
**US-A- 3 496 136**

**CHEMICAL ABSTRACTS, Band 99, Nr. 22, 28. November 1983, Seite 40, Zusammenfassung Nr. 176847q, Columbus, Ohio, US;**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel(CH)**

(72) Erfinder: **Wehner, Wolfgang, Dr., Wetzbach 34, D-6144 Zwingenberg(DE)**

**EP 0 255 801 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft mit Anilinotriazinen stabilisierte chlorhaltige Polymerisate sowie neue Anilinotriazine und deren Verwendung zum Stabilisieren von chlorhaltigen Polymerisaten gegen den schädigenden Einfluss von Licht und/oder Wärme.

In der US-Patentschrift 3 714 114 wird die Verwendung von substituierten Melaminen zum Stabilisieren von pigmentiertem Polyvinylchlorid beschrieben. Aus der US-Patentschrift 3 496 136 ist ferner eine Stabilisatorzusammensetzung bekannt, welche a) ein substituiertes Melamin und b) eine UV-Licht absorbierende Substanz enthält.

Die Herstellung von 2-Anilino-4,6-bis(2-hydroxyethylamino)-1,3,5-triazin und von ähnlichen Verbindungen wurde bereits 1950 von D.F. Walker et al. im "Journal of the American Pharmaceutical Association, Vol. 39, 393-396" und 1951 von D.W. Kaiser et al. im "Journal of the American Chemical Society, Vol. 73, pp. 2984-2986 beschrieben.

Gegenstand der Erfindung ist ein chlorhaltiges Polymerisat, enthaltend mindestens eine Verbindung der Formel I,

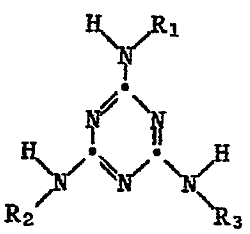

(I)

worin $R_1$ Phenyl oder eine Gruppe der Formel II bedeutet,

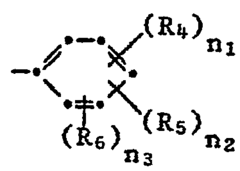

(II)

worin, $R_4$, $R_5$ und $R_6$ unabhängig voneinander Chlor, Brom, Cyan, Methoxycarbonyl, Ethoxycarbonyl, Carbamoyl, Hydroxy, Methoxy, Ethoxy oder $C_1$-$C_{18}$-Alkyl sind und $n_1$, $n_2$ und $n_3$ 0, 1, 2 oder 3 bedeuten, mit der Bedingung, dass die Summe $n_1+n_2+n_3$ eine ganze Zahl von 1 bis 3 ist, $R_2$ und $R_3$ unabhängig voneinander $C_2$-$C_4$-Hydroxyalkyl bedeuten.

Die Anilinotriazine der Formel I besitzen einen besonders günstigen Einfluss auf die Farbstabilität von chlorhaltigen Polymerisaten bei der thermoplastischen Verarbeitung und bei Belichtung.

$R_1$ als eine Gruppe der Formel II bedeutet beispielsweise o-, m- oder p-Chlorphenyl, 2,3-Dichlorphenyl, 2,4-Dichlorphenyl, 2,5-Dichlorphenyl, 2,6-Dichlorphenyl, 3,4-Dichlorphenyl, 2,4,5-Trichlorphenyl, 2,4,6-Trichlorphenyl, o-, m- oder p-Hydroxyphenyl, o-, m- oder p-Methylphenyl, 2,3-dimethylphenyl, 2,4-Dimethylphenyl, 2,5-Dimethylphenyl, 2,6-Dimethylphenyl, 3,4-Dimethylphenyl, 3,5-Dimethylphenyl, 2-Methyl-6-ethylphenyl, 2-Methyl-4-tert-butylphenyl, 2-Ethylphenyl, 2,6-Diethylphenyl, 2,6-Diethyl-4-methylphenyl, 2,6-Diisopropylphenyl, 4-tert-Butylphenyl, o-, m- oder p-(n- Dodecyl)phenyl, 2-Chlor-6-methylphenyl, 3-Chlor-2-methylphenyl, 3-Chlor-4-methylphenyl, 4-Chlor-2-methylphenyl, 5-Chlor-2-methylphenyl, 2,6-Dichlor-3-methylphenyl, 2-Hydroxy-4-methylphenyl, 3-Hydroxy-4-methylphenyl, o-, m- oder p- Methoxyphenyl, o- oder p-Ethoxyphenyl, 2,4-Dimethoxyphenyl, 2,5-Dimethoxyphenyl, 2,5-Diethoxyphenyl, 2-Methoxy-5-methylphenyl, 4-Methoxy-2-methylphenyl, 3-Chlor-4-methoxyphenyl, 3-Chlor-6-methoxyphenyl, 3-Chlor-4,6-di methoxyphenyl oder 4-Chlor-2,5-dimethoxyphenyl. Bevorzugt sind Phenylreste, die mindestens eine Methoxygruppe enthalten. Besonders bevorzugt sind o-, m- oder p-Chlorphenyl und o-, m- oder p-Hydroxyphenyl.

$R_2$ und $R_3$ bedeuten als $C_2$-$C_4$-Hydroxyalkyl beispielsweise 2-Hydroxyethyl, 2-Hydroxypropyl, 3-Hydroxypropyl, 1-Hydroxymethylethyl, 3-Hydroxybutyl, 1,1-Dimethyl-2-hydroxyethyl oder 4-Hydroxybutyl. 2-Hydroxyethyl ist bevorzugt.

Ein bevorzugter Gegenstand der Erfindung ist ein chlorhaltiges Polymerisat, enthaltend mindestens eine Verbindung der Formel I, worin $R_1$ Phenyl oder eine Gruppe der Formel II bedeutet, worin $R_4$, $R_5$ und $R_6$ unabhängig voneinander Chlor, Brom, Hydroxy, Methoxy, Ethoxy oder $C_1$-$C_{18}$-Alkyl sind.

Von Interesse ist ein chlorhaltiges Polymerisat, enthaltend mindestens eine Verbindung der Formel I, worin $R_1$ Phenyl oder eine Gruppe der Formel II bedeutet, worin $R_4$, $R_5$ und $R_6$ unabhängig voneinander Chlor, Hydroxy, Methoxy, Ethoxy oder $C_1$-$C_{12}$-Alkyl sind.

Bevorzugt ist ein chlorhaltiges Polymerisat, enthaltend mindestens eine Verbindung der Formel I, worin $R_1$ Phenyl oder eine Gruppe der Formel II bedeutet, worin $R_4$, $R_5$ und $R_6$ unabhängig voneinander Chlor, Hydroxy, Methoxy oder Methyl sind.

Ein weiterer bevorzugter Gegenstand dieser Erfindung ist ein chlorhaltiges Polymerisat, enthaltend mindestens eine Verbindung der Formel I, worin $R_1$ Phenyl oder eine Gruppe der Formel II bedeutet, worin $R_4$ $R_5$ und $R_6$ unabhängig voneinander Chlor oder Hydroxy sind.

Ebenfalls bevorzugt ist ein chlorhaltiges Polymerisat, enthaltend mindestens eine Verbindung der Formel I, worin $R_1$ Phenyl oder eine Gruppe der Formel II bedeutet, worin die Reste $R_4$, $R_5$ und $R_6$ gleich sind.

Von besonderem Interesse ist ein chlorhaltiges Polymerisat, enthaltend mindestens eine Verbindung der Formel I, worin $R_1$ Phenyl oder eine Gruppe der Formel II bedeutet, worin $n_1$ und $n_2$ 0 bedeuten und $n_3$ 1 ist.

Besonders bevorzugt ist ein chlorhaltiges Polymerisat, enthaltend mindestens eine Verbindung der Formel I, worin $R_1$ Phenyl bedeutet.

Weiterhin von Interesse ist ein chlorhaltiges Polymerisat, enthaltend mindestens eine Verbindung der Formel I, worin $R_2$ und $R_3$ 2-Hydroxyethyl bedeuten.

Bei den chlorhaltigen Polymerisaten handelt es sich bevorzugt um Vinylchloridhomopolymere oder -copolymere. Weiterhin bevorzugt sind Suspensions- und Massepolymere sowie Emulsionspolymere. Als Comonomere für die Copolymerisate kommen z.B. in Frage: Vinylacetat, Vinylidenchlorid, Transdichlorethen, Ethylen, Propylen, Butylen, Maleinsäure, Acrylsäure, Fumarsäure, Itaconsäure. Weitere geeignete chlorhaltige Polymere sind nachchloriertes PVC und chlorierte Polyolefine, ferner Pfropfpolymerisate von PVC mit EVA, ABS und MBS.

Die Verbindungen der Formel I eignen sich insbesondere zum Stabilisieren von Polyvinylchlorid.

Es ist vorteilhaft, wenn die erfindungsgemässen chlorhaltigen Polymerisate zusätzlich mindestens einen herkömmlichen Thermostabilisator enthalten. Solche Stabilisatoren sind beispielsweise in "Pure & Appl. Chem., 49, 627-648 (1977)" genannt. Thermostabilisatoren sind zum Beispiel Me(II)-carboxylate und Me(II)-phenolate, wobei Me(II) Mg, Ca, Ba, Zn oder Cd bedeuten kann und Carboxylat z.B. Stearat, Oleat, Laurat, Palmitat, Behenat, Benzoat, Hydroxystearat oder 2-Ethylhexanoat sein kann, sowie Alkylphenolate, insbesondere Nonylphenolat, und Organozinnverbindungen. Als Organozinnverbindungen seien zum Beispiel genannt:
n-Octylzinn-tris-[isooctylthioglykolat], Di-n-octylzinn-bis[isooctylthioglykolat], Dibutylzinnsulfid, Dibutylzinn-thioglykolat oder Butylzinnsulfid sowie Methylzinn-tris[alkylthioglykolat], n-Butylzinn-tris[alkyl thioglykolat], n-Butoxycarbonylethylzinn-tris[alkylthioglykolat], Dimethylzinn-bis[alkylthioglykolat],
Di-n-butylzinn-bis[alkylthioglykolat], Bis[n-butoxycarbonylethyl]-zinn-bis[alkylthioglykolat],
Methylzinn-tris[alkylthiopropionat], n-Butylzinn-tris-[alkylthiopropionat], n-Butoxycarbonylethylzinn-tris[alkylthiopropionat], Dimethylzinn-bis[alkylthiopropionat], Di-n-butylzinn-bis[alkylthiopropionat],
Bis[n-butoxycarbonylethyl]-zinn-bis[alkylthiopropionat], wobei Alkyl beispielsweise 2-Ethylhexyl,
Dodecyl, Tridecyl oder Tetradecyl bedeutet, und auch Organozinncarboxylate, insbesondere Maleinate oder Halbestermaleinate.

Ein weiterer Gegenstand der Erfindung ist daher ein chlorhaltiges Polymerisat, enthaltend mindestens eine Verbindung der Formel I und zusätzlich mindestens ein Me(II)-carboxylat und/oder Me(II)-phenolat oder- alkylphenolat, wobei Me(II) Mg, Ca, Ba, Zn oder Cd ist und Carboxylat das Anion einer Carbonsäure mit 7 bis 20 C-Atomen bedeutet.

Bei den $C_7$-$C_{20}$-Carbonsäuren handelt es sich beispielsweise um Benzoesäure, p-tert-Butylbenzoesäure oder aliphatische Carbonsäuren, insbesondere Octansäure, Dodecansäure, Stearinsäure oder Oelsäure.

Von besonderem Interesse sind chlorhaltige Polymerisate, enthaltend eine Verbindung der Formel I und zusätzlich ein Gemisch von Barium Zink-, Calcium/Zink- oder Magnesium/Zink Carboxylaten.

Ebenfalls ein Gegenstand der Erfindung ist die Verwendung einer Verbindung der Formel I zum Stabilisieren von einem chlorhaltigen Polymerisat gegen den schädigenden Einfluss von Licht und/oder Wärme.

Die Einarbeitung der Stabilisatorkomponenten in das chlorhaltige Polymerisat erfolgt am günstigsten wie üblich auf einem 2-Walzenstuhl bei Temperaturen zwischen 150 und 200°C. Im allgemeinen lässt sich eine genügende Homogenisierung innerhalb von 5 bis 15 Minuten erreichen. Die Zugabe der Komponenten kann einzeln oder gemeinsam als Vorgemisch erfolgen. Als zweckmässig hat sich ein flüssiges Vorgemisch erwiesen, d.h. es wird in Gegenwart von indifferenten Lösungsmitteln und/oder Weichmachern gearbeitet.

Die Me(II)-carboxylate, Me(II)-phenolate oder Me(II)-alkylphenolate können in dem zu stabilisierenden Material in einer dem Fachmann bekannten Konzentration vorliegen, wie zum Beispiel in Mengen von 0,05 bis 5 Gew.-%.

Die Verbindung der Formel I wird beispielsweise in Mengen von 0.05 bis 10, bevorzugt 0,1 bis 5 und besonders bevorzugt 0,1 bis 2 Gew.-% in das chlorhaltige Polymerisat eingearbeitet.

Die Angabe Gew.-% bezieht sich jeweils auf das zu stabilisierende Material.

Zusätzlich können die chlorhaltigen Polymerisate in üblichen Mengen herkömmliche PVC-Stabilisatoren enthalten, wie beispielsweise Epoxyverbindungen, vorzugsweise epoxidierte Fettsäureester, wie

epoxydiertes Sojabohnenöl, phenolische Antioxidantien oder Phosphite, wie z.B. Trioctyl-, Tridecyl-, Tridodecyl-, Tritetradecyl-,Tristearyl-, Trioleyl-, Triphenyl-, Trikresyl-, Tris[p-nonyl-phenyl]- oder Tricyclohexylphosphit und besonders bevorzugt Aryldialkyl- sowie Alkyl-diarylphosphite wie z.B. Phenyl-didecyl-, Nonylphenyl-didecyl-, (2,4-Di-tert-butylphenyl)-didodecyl- oder (2,6-Di-tert-butylphenyl)-didodecylphosphit.

Je nach Verwendungszweck der Polymerisate können vor oder bei der Einarbeitung der Stabilisatoren auch weitere Zusätze eingearbeitet werden, wie zum Beispiel Gleitmittel (bevorzugt Montanwachse oder Glycerinester), Fettsäureester, Paraffine, Weichmacher (wie z.B. Phthalsäure-, Phosphorsäure-, Adipinsäure-, Azelainsäure-, Sebacinsäure- und Citronensäureester), Füllstoffe, Russ, Asbest, Kaolin, Talk, Glasfasern, Modifikatoren (wie etwa Schlagzäh-Zusätze), optischen Aufheller, Pigmente, Lichtschutzmittel, UV-Absorber, Flammschutzmittel oder Antistatika.

Ein weiterer Gegenstand der Erfindung sind die neuen Verbindungen der Formel IA,

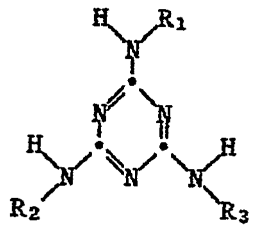

(IA)

worin $R_1$ eine Gruppe der Formel II ist,

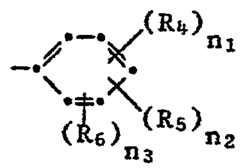

(II)

worin $R_4$, $R_5$ und $R_6$ unabhängig voneinander Chlor, Brom, Cyan, Methoxycarbonyl, Ethoxycarbonyl, Carbamoyl, Hydroxy, Methoxy, Ethoxy oder $C_1$-$C_{18}$-Alkyl sind und $n_1$, $n_2$ und $n_3$ 0, 1, 2 oder 3 bedeuten, mit der Bedingung, dass die Summe $n_1+n_2+n_3$ eine ganze Zahl von 1 bis 3 ist, $R_2$ und $R_3$ unabhängig voneinander $C_2$-$C_4$-Hydroxyalkyl bedeuten.

Ein bevorzugter Gegenstand dieser Erfindung sind auch Verbindungen der Formel IA, worin $R_4$, $R_5$ und $R_6$ unabhängig voneinander Chlor, Brom, Hydroxy, Methoxy, Ethoxy oder $C_1$-$C_{18}$-Alkyl sind.

Bevorzugt sind Verbindungen der Formel IA, worin $R_4$, $R_5$ und $R_6$ unabhängig voneinander Chlor, Hydroxy, Methoxy, Ethoxy oder $C_1$-$C_{12}$-Alkyl, insbesondere Chlor oder Hydroxy, sind.

Ebenfalls bevorzugt sind Verbindungen der Formel IA, worin $R_4$, $R_5$ und $R_6$ unabhängig voneinander Chlor, Hydroxy, Methoxy oder Methyl sind. Besonders bevorzugt sind die Reste $R_4$, $R_5$ und $R_6$ gleich. Von Interesse sind Verbindungen der Formel IA, worin $n_1$ und $n_2$ 0 sind und $n_3$ 1 bedeutet.

Von besonderem Interesse sind Verbindungen der Formel IA, worin $R_2$ und $R_3$ 2-Hydroxyethyl bedeuten.

Beispiele für Verbindungen der Formel IA sind:
2,4-Bis(2-hydroxyethylamino)-6-(2-chloranilino)-1,3,5-triazin,
2,4-Bis(2-hydroxyethylamino)-6-(3-chloranilino)1,3,5-triazin,
2,4-Bis(2-hydroxyethylamino)-6-(4-chloranilino)-1,3,5-triazin,
2,4-Bis(2-hydroxyethylamino)-6-(2-methoxyanilino)-1,3,5-triazin,
2,4-Bis(2-hydroxyethylamino)-6-(3-methoxyanilino)-1,3,5-triazin,
2,4-Bis(2-hydroxyethylamino)-6-(4-methoxyanilino)-1,3,5-triazin,
2,4-Bis(2-hydroxyethylamino)-6-(2-ethoxyanilino)-1,3,5-triazin,
2,4-Bis(2-hydroxyethylamino)-6-(3-ethoxyanilino)-1,3,5-triazin,
2,4-Bis(2-hydroxyethylamino)-6-(2,4-dimethoxyanilino)-1,3,5-triazin,
2,4-Bis(2-hydroxyethylamino)-6-(2,5-dimethoxyanilino)-1,3,5-triazin,
2,4-Bis(2-hydroxyethylamino)-6-(3,4-dimethoxyanilino)-1,3,5-triazin,
2,4-Bis(2-hydroxyethylamino)-6-(3,5-dimethoxyanilino)-1,3,5-triazin,
2,4-Bis(2-hydroxyethylamino)-6-(4-ethoxyanilino)-1,3,5-triazin,
2,4-Bis(2-hydroxyethylamino)-6-(2-n-dodecylanilino)-1,3,5-triazin,
2,4-Bis(2-hydroxyethylamino)-6-(3-n-dodecylanilino)-1,3,5-triazin,
2,4-Bis(2-hydroxyethylamino)-6-(4-n-dodecylanilino)-1,3,5-triazin,
2,4-Bis(2-hydroxyethylamino)-6-(2-hydroxyanilino)-1,3,5-triazin,
2,4-Bis(2-hydroxyethylamino)-6-(3-hydroxyanilino)-1,3,5-triazin,

2,4-Bis(2-hydroxyethylamino)-6-(4-hydroxyanilino)-1,3,5-triazin,
2,4-Bis(2-hydroxyethylamino)-6-(3-hydroxy-4-methoxyanilino)-1,3,5-triazin,
2,4-Bis(2-hydroxyethylamino)-6-(2-methylanilino)-1,3,5-triazin,
2,4-Bis(2-hydroxyethylamino)-6-(3-methylanilino)-1,3,5-triazin,
2,4-Bis(2-hydroxyethylamino)-6-(4-methylanilino)-1,3,5-triazin,
2,4-Bis(2-hydroxyethylamino)-6-(2-ethylanilino)-1,3,5-triazin,
2,4-Bis(2-hydroxyethylamino)-6-(3-ethylanilino)-1,3,5-triazin,
2,4-Bis(2-hydroxyethylamino)-6-(4-ethylanilino)-1,2,5-triazin,
2,4-Bis(2-hydroxyethylamino)-6-(2-propylanilino)-1,3,5-triazin,
2,4-Bis(2-hydroxyethylamino)-6-(4-propylanilino)-1,3,5-triazin,
2,4-Bis(2-hydroxyethylamino)-6-(4-n-butylanilino)-1,3,5-triazin,
2,4-Bis(2-hydroxyethylamino)-6-(4-tert-butylanilino)-1,3,5-triazin,
2,4-Bis(2-hydroxyethylamino)-6-(4-octylanilino)-1,3,5-triazin.

Besonders bevorzugt sind 2,4-Bis(2-hydroxyethylamino)-6-(3-hydroxyanilino)-1,3,5-triazin, 2,4-Bis(2-hydroxyethylamino)-6-(2-chloranilino)-1,3,5-triazin, 2,4-Bis(2-hydroxyethylamino)-6-(3-chloranilino)-1,3,5-triazin, 2,4-Bis(2-hydroxyethylamino)-6-(4-chloranilino)-1,3,5-triazin und 2,4-Bis(2-hydroxyethylamino)-6-(3-methoxyanilino)-1,3,5-triazin.

Die Verbindungen der Formeln I und IA können auf an sich bekannte Weise, z.B. wie in einem Artikel von D.W. Kaiser et al. im "Journal of the American Chemical Society, 73, 2984 (1951)" beschrieben, hergestellt werden, indem man eine Verbindung der Formel III,

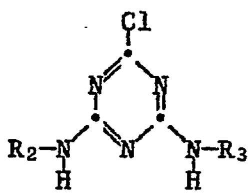

(III)

worin $R_2$ und $R_3$ die oben angegebenen Bedeutungen haben, mit Anilin oder einem Anilinderivat der Formel IV,

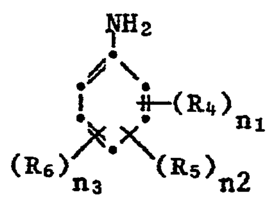

(IV)

worin $R_4$, $R_5$, $R_6$, $n_1$, $n_2$ und $n_3$ die in den vorangehenden Ausführungen angegebenen Definitionen besitzen, in einer wässrigen Lösung in Gegenwart von NaOH umsetzt. Es ist vorteilhaft, die Reaktion bei einer Temperatur zwischen 80 und 100°C durchzuführen.

Die Verbindungen der Formel III können in Analogie zu bekannten Verfahren, zum Beispiel durch Umsetzung von Cyanurchlorid (gelöst in Aceton) mit $H_2NR_2$ bzw. $H_2NR_3$ in Gegenwart von Natriumhydroxid, hergestellt werden. Als Reaktionsmedium wird zweckmässigerweise eine wässrige Lösung verwendet. Die Temperatur liegt bevorzugt zwischen 0 und 30°C.

Die Anilinderivate der Formel IV sind bekannt (zum grössten Teil im Handel erhältlich) und können ebenfalls in Analogie zu bekannten Verfahren hergestellt werden.

Die folgenden Beispiele erläutern die Erfindung weiter. Teile und Prozent beziehen sich darin, ebenso wie in der übrigen Beschreibung, auf das Gewicht, sofern nichts anderes angegeben ist.

Beispiel 1:

Herstellung von 2,4-Bis(2-hydroxyethylamino)-6-anilino-1,3,5-triazin

In einem 500 ml Kolben mit Rührer, Rückflusskühler und Thermometer werden 33 g Eis und 67 ml Wasser vorgelegt. Unter heftigem Rühren lässt man eine Lösung von 31,0g (0,168 Mol) Cyanurchlorid in 75 ml Aceton zutropfen und erhält eine feine Suspension. Zu dieser gibt man tropfenweise, ebenfalls unter Rühren, 20,5 g (0,336 Mol) Ethanolamin und lässt die nun nahezu klare Lösung auf Raumtemperatur kommen. Anschliessend wird eine Lösung von 13,4 g Natriumhydroxid in 50 ml Wasser so zugetropft, dass der pH-Wert 7 nicht übersteigt. Nach vollendeter Zugabe beträgt der pH-Wert 6,85. Der entstandene Niederschlag wird abfiltriert und chloridfrei gewaschen. Das noch feuchte Produkt (2,4-Bis(2-hydroxyethylamino)-6-chlor-1,3,5-triazin) wird in 200 ml Wasser suspendiert. Unter Rühren tropft man 14,7

g (0,158 Mol) Anilin zu und erhitzt auf 90°C. Dabei entsteht eine klare Lösung. Man gibt nun 6,0 g Natriumhydroxid gelöst in 50 ml Wasser zu und erhitzt die Reaktionslösung 3 Stunden am Rückfluss. Anschliessend werden zu der noch heissen, leicht trüben Lösung 75 ml Isopropanol gegeben. Die nun klare Lösung lässt man langsam auf 0°C abkühlen, wobei sich ein dicker Kristallbrei abscheidet. Der Niederschlag wird abgesaugt, mit wenig kaltem Wasser chloridfrei gewaschen und getrocknet. Das Produkt besitzt einen Schmelzpunkt von 120-122°C. Die Ausbeute beträgt 39,2 g (=90,1% der Theorie).

Wird das Produkt aus 200 ml Isopropanol umkristallisiert, so erhöht sich der Schmelzpunkt auf 128-130°C.

Beispiel 2:

Herstellung von 2,4-Bis(2-hydroxyethylamino)-6-(3-hydroxyanilino)-1,3,5-triazin

In einem 2 l-Kolben mit Rührer, Tropftrichter, Rückflusskühler und Thermometer werden 70,1 g (0,3 Mol) 2,4-Bis(2-hydroxyethylamino)-6-chlor-1,3,5-triazin, 32,8 g (0,3 Mol) 3-hydroxyanilin und 400 ml Wasser vorgelegt. Das Gemisch wird unter Rühren am Rückfluss erhitzt. Dabei wird eine Lösung von 12 g Natriumhydroxid in 60 ml Wasser so langsam zugetropft, das ein schwach alkalisches Reaktionsgemisch resultiert. Anschliessend gibt man 300 ml Wasser und zur Farbaufhebung Aktivkohle zu. Dann wird die heisse Lösung filtriert und abgekühlt. Die erhaltenen, nahezu farblosen Kristalle werden abgesaugt, mit wenig Wasser chloridfrei gewaschen und getrocknet. Das Produkt besitzt einen Schmelzpunkt von 142-143°C. Die Ausbeute beträgt 70,8 g (=77 % der Theorie).

Beispiel 3

Herstellung von 2,4-Bis(2-hydroxyethylamino)-6-(2-chloranilino)-1,3,5-triazin

In einem 250 ml-Kolben mit Rührer, Tropftrichter, Rückflusskühler und Thermometer werden 6,8 g (0,053 Mol) 2,4-Bis(2-hydroxyethylamino)-6-chlor-1,3,5-triazin, 11,7 g (0,05 Mol) 2-Chloranilin und 85 ml Wasser vorgelegt. Das Gemisch wird unter Rühren auf 90°C erhitzt, wobei eine klare Lösung resultiert. Zu dieser Mischung tropft man eine Lösung von 2 g Natriumhydroxid in 15 ml Wasser und erhitzt das Gemisch 3 Stunden am Rückfluss. Nach dem Abkühlen liegt ein 2-phasig-wässriges System vor. Die überstehende anorganische Phase wird verworfen. Die untere Phase wird mit Toluol ausgekocht und man erhält das Produkt im Rückstand. Der Schmelzpunkt liegt bei 108-110°C und nach Umfällen mit Essigester-Petrolether bei 112-114°C. Die Ausbeute beträgt 82 % der Theorie.

Beispiel 4

Herstellung von 2,4-Bis(2-hydroxyethylamino)-6-(3-chloranilino)-1,3,5-triazin

Die Herstellung erfolgt in Analogie zu Beispiel 3. An Stelle von 2-Chloranilin wird 3-Chloranilin eingesetzt. Das Produkt besitzt einen Schmelzpunkt von 134-136°C. Die Ausbeute beträgt 79 % der Theorie.

Beispiel 5

Herstellung von 2,4-Bis(2-hydroxyethylamino)-6-(4-chloranilino)-1,3,5-triazin

Die Herstellung erfolgt in Analogie zu Beispiel 3. An Stelle von 2-Choranilin wird 4-Chloranilin eingesetzt und nach erfolgter Umsetzung wird die untere Phase mit Isopropanol (Umkristallisation) statt mit Toluol behandelt. Das Produkt besitzt einen Schmelzpunkt von 150-152°C. Die Ausbeute beträgt 72 % der Theorie.

Beispiele 6-12:

Die Verbindungen der Formel

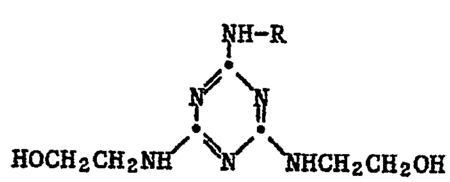

werden in Analogie zu Beispiel 3 hergestellt. Nach beendeter NaOH-Zugabe und Erhitzen am Rückfluss wird der resultierende Feststoff abgetrennt, mit Wasser gewaschen und bis zur Gewichtskonstanz getrocknet.

| Beispiel | R | Ausbeute [% der Theorie] | Schmelzpunkt [°C] | Farbe |
|---|---|---|---|---|
| 6 | 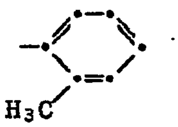 | 69 | 80 | beige |
| 7 | 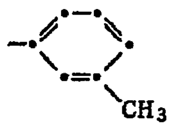 | 90 | 80 | beige |
| 8 | 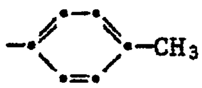 | 81 | 140 | farblos |
| 9 | 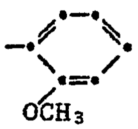 | 84 | 130 | farblos |
| 10 | 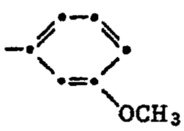 | 74[x)] | 185 | farblos |
| 11 | 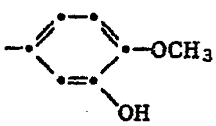 | 60[xx)] | 160 | beige |
| 12 | 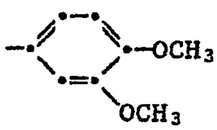 | 44[xxx)] | 125 | farblos |

x) nach Umfällen aus Aceton/Petrolether

xx) nach Umfällen aus Isopropanol/Petrolether

xxx) nach Aufarbeitung

Beispiel 13

Die unten angegebene Rezeptur wird auf einem Mischwalzwerk 5 Minuten bei 180°C gewalzt. Vom gebildeten 0,3 mm dicken Walzfell werden Folienmuster in einem Testofen bei 180°C thermisch belastet. Der Yellowness Index (YI) der Proben wird in regelmässigen Zeitabständen nach ASTM D 1925-70 bestimmt. Die Ergebnisse sind in Tabelle 1 aufgeführt.

Rezeptur:

S-PVC (K-Wert 64) 100 Teile
Epoxidiertes Sojabohnenöl 3 Teile
Didecyl-phenylphosphit 0,55 Teile
Kalziumstearat 0,35 Teile
Zinkstearat 0,15 Teile
Additiv gemäss Tabelle 1 0,3 Teile

Tabelle 1:

| Additiv | YI nach Belastungszeit in Minuten | | | | |
|---|---|---|---|---|---|
| | 0 | 5 | 10 | 15 | 20 |
| Ohne | 20,4 | 30,9 | 34,3 | 34,9 | 30,3 |
| A | 2,8 | 4,4 | 5,8 | 8,0 | 14,1 |
| B | 4,7 | 6,4 | 8,8 | 11,6 | 14,4 |
| C | 3,7 | 5,0 | 6,9 | 9,8 | 15,5 |
| D | 2,6 | 4,1 | 5,8 | 9,3 | 12,7 |
| E | 2,7 | 4,3 | 5,1 | 8,2 | 10,6 |

A: 2,4-Bis(2-hydroxyethylamino)-6-anilino-1,3,5-triazin

B: 2,4-Bis(2-hydroxyethylamino)-6-(3-hydroxyanilino)-1,3,5-triazin

C: 2,4-Bis(2-hydroxyethylamino)-6-(2-chloranilino)-1,3,5-triazin

D: 2,4-Bis(2-hydroxyethylamino)-6-(3-chloranilino)-1,3,5-triazin

E: 2,4-Bis(2-hydroxyethylamino)-6-(4-chloranilino)-1,3,5-triazin

Beispiel 14:

Die unten angegebene Rezeptur wird auf einem Mischwalzwerk 5 Minuten bei 190°C gewalzt. Vom gebildeten 0,3 mmm dicken Walzfell werden Folienmuster in einem Testofen bei 180°C thermisch belastet. Der Yellowness Index (YI) der Proben wird in regelmässigen Zeitabständen nach ASTM D 1925-70 bestimmt. Die Ergebnisse sind in Tabelle 2 aufgeführt.

Rezeptur:

S-PVC (K-Wert 64) 100 Teile
Dioctylphthalat (Weichmacher) 17 Teile
Epoxidiertes Sojabohnenöl 3 Teile
Zink-dioleat 0,5 Teile
Barium-bis[p-t-butylbenzoat] 0,54 Teile
Didecyl-phenylphosphit 0,64 Teile
2,6-Di-t-butyl-4-methylphenol 0,06 Teile
Oelsäure 0,02 Teile
Additiv gemäss Tabelle 2 0,2 Teile

Tabelle 2:

| Additiv | YI nach Belastungszeit in Minuten | | | | | |
|---|---|---|---|---|---|---|
| | 0 | 10 | 20 | 30 | 40 | 50 |
| Ohne | 8,4 | 15,2 | 23,2 | 28,2 | 26,4 | 26,9 |
| F | 2,8 | 4,2 | 5,7 | 5,9 | 9,3 | 12,4 |
| G | 4,2 | 6,5 | 8,0 | 9,9 | 10,9 | 16,4 |
| H | 3,5 | 4,4 | 5,9 | 7,1 | 10,5 | 11,5 |

F: 2,4-Bis(2-hydroxyethylamino)-6-(4-methylanilino)-1,3,5-triazin

G: 2,4-Bis(2-hydroxyethylamino)-6-(2-methoxyanilino)-1,3,5-triazin

H: 2,4-Bis(2-hydroxyethylamino)-6-(3-methoxyanilino)-1,3,5-triazin

## Patentansprüche

1. Ein chlorhaltiges Polymerisat, enthaltend mindestens eine Verbindung der Formel I,

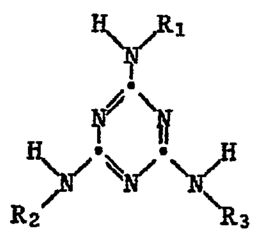

(I)

worin $R_1$ Phenyl oder eine Gruppe der Formel II bedeutet,

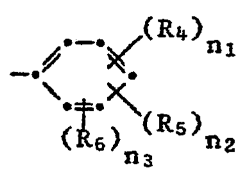

(II)

worin $R_4$, $R_5$ und $R_6$ unabhängig voneinander Chlor, Brom, Cyan, Methoxycarbonyl, Ethoxycarbonyl, Carbamoyl, Hydroxy, Methoxy, Ethoxy oder $C_1$-$C_{18}$-Alkyl sind und $n_1$, $n_2$ und $n_3$ 0, 1, 2 oder 3 bedeuten, mit der Bedingung, dass die Summe $n_1+n_2+n_3$ eine ganze Zahl von 1 bis 3 ist, $R_2$ und $R_3$ unabhängig voneinander $C_2$-$C_4$-Hydroxyalkyl bedeuten.

2. Ein chlorhaltiges Polymerisat gemäss Anspruch 1, worin $R_1$ Phenyl oder eine Gruppe der Formel II bedeutet, worin $R_4$, $R_5$ und $R_6$ unabhängig voneinander Chlor, Brom, Hydroxy, Methoxy, Ethoxy oder $C_1$-$C_{18}$-Alkyl sind.

3. Ein chlorhaltiges Polymerisat gemäss Anspruch 1, worin $R_1$ Phenyl oder eine Gruppe der Formel II bedeutet, worin $R_4$, $R_5$ und $R_6$ unabhängig voneinander Chlor, Hydroxy, Methoxy, Ethoxy oder $C_1$-$C_{12}$-Alkyl sind.

4. Ein chlorhaltiges Polymerisat gemäss Anspruch 1, worin $R_1$ Phenyl oder eine Gruppe der Formel II bedeutet, worin $R_4$, $R_5$ und $R_6$ unabhängig voneinander Chlor, Hydroxy, Methoxy oder Methyl sind.

5. Ein chlorhaltiges Polymerisat gemäss Anspruch 1, worin $R_1$ Phenyl oder eine Gruppe der Formel II bedeutet, worin die Reste $R_4$, $R_5$ und $R_6$ gleich sind.

6. Ein chlorhaltiges Polymerisat gemäss Anspruch 1, worin $R_1$ Phenyl oder eine Gruppe der Formel II bedeutet, worin $n_1$ und $n_2$ 0 bedeuten und $n_3$ 1 ist.

7. Ein chlorhaltiges Polymerisat gemäss Anspruch 1, worin $R_1$ Phenyl bedeutet.

8. Ein chlorhaltiges Polymerisat gemäss Anspruch 1, worin $R_2$ und $R_3$ 2-Hydroxyethyl bedeuten.

9. Ein chlorhaltiges Polymerisat gemäss Anspruch 1, enthaltend zusätzlich mindestens ein Me(II)-carboxylat und/oder ME(II)-phenolat oder -alkylphenolat, wobei Me(II) Mg, Ca, Ba, Zn oder Cd ist und Carboxylat das Anion einer Carbonsäure mit 7 bis 20 C-Atomen bedeutet.

10. Ein chlorhaltiges Polymerisat gemäss Anspruch 9, dadurch gekennzeichnet, dass es sich bei dem Me(II)-carboxylat um ein Gemisch aus Barium/Zink-, Calcium/Zink- oder Magnesium/Zink-Carboxylat handelt.

11. Ein chlorhaltiges Polymerisat gemäss Anspruch 1, dadurch gekennzeichnet, dass es sich bei dem chlorhaltigen Polymerisat um Polyvinylchlorid handelt.

12. Verbindungen der Formel IA,

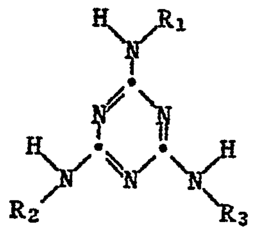

(IA)

worin $R_1$ eine Gruppe der Formel II ist,

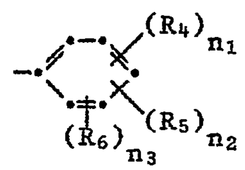

(II)

worin $R_4$, $R_5$ und $R_6$ unabhängig voneinander Chlor, Brom, Cyan, Methoxycarbonyl, Ethoxycarbonyl, Carbamoyl, Hydroxy, Methoxy, Ethoxy oder $C_1$-$C_{18}$-Alkyl sind und $n_1$, $n_2$ und $n_3$ 0, 1, 2 oder 3 bedeuten, mit der Bedingung, dass die Summe $n_1+n_2+n_3$ eine ganze Zahl von 1 bis 3 ist, $R_2$ und $R_3$ unabhängig voneinander $C_2$-$C_4$-Hydroxyalkyl bedeuten.

13. Die Verbindungen
2,4-Bis(2-hydroxyethylamino)-6-(3-hydroxyanilino)1,3,5-triazin,
2,4-Bis(2-hydroxyethylamino)-6-(2-chloranilino)-1,3,5-triazin,
2,4-Bis(2-hydroxyethylamino)-6-(3-chloranilino)-1,3,5-triazin,
2,4-Bis(2-hydroxyethylamino)-6-(4-chloranilino)-1,3,5-triazin und
2,4-Bis(2-hydroxyethylamino)-6-(3-methoxyanilino)-1,3,5-triazin gemäss Anspruch 12.

14. Verwendung der im Anspruch 1 definierten Verbindungen der Formel I zum Stabilisieren von chlorhaltigen Polymerisaten gegen den schädigenden Einfluss von Licht und/oder Wärme.

**Revendications**

1. Polymère chloré qui contient au moins un composé répondant à la formule I

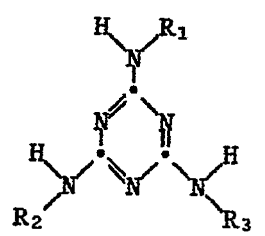

(I)

dans laquelle $R_1$ représente un radical phényle ou un radical répondant à la formule II:

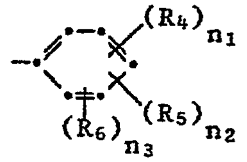

(II)

dans laquelle $R_4$, $R_5$ et $R_6$ représentent chacun, indépendamment les uns des autres, le chlore, le brome, un radical, cyano, méthoxycarbonyle, éthyoxycarbonyle, carbamoyle, hydroxy, méthoxy ou éthoxy ou un radical alkyle en $C_1$–$C_{18}$, $n_1$, $n_2$ et $n_3$ représentent chacun un nombre égal à 0, à 1, à 2 ou à 3 avec la condition que la somme ($n_1+n_2+n_3$) soit égale à un nombre de 1 à 3, et $R_2$ et $R_3$ représentent chacun, indépendamment l'un de l'autre, un radical hydroxy-alkyle contenant de 2 à 4 atomes de carbone.

2. Polymère chloré selon la revendication 1 dans lequel $R_1$ représente un phényle ou un radical de formule II dans lequel $R_4$, $R_5$ et $R_6$ représentent chacun, indépendamment les uns des autres, le chlore, le brome, un hydroxy, un méthoxy, un éthoxy ou un alkyle en $C_1$–$C_{18}$.

3. Polymère chloré selon la revendication 1 dans lequel $R_1$ représente un phényle ou un radical de formule II dans lequel $R_4$, $R_5$ et $R_6$ représentent chacun, indépendamment les uns des autres, un hydroxy, un méthoxy, un éthoxy ou un alkyle en $C_1$–$C_{12}$.

4. Polymère chloré selon la revendication 1 dans lequel $R_1$ représente un phényle ou un radical de formule II dans lequel $R_4$, $R_5$ et $R_6$ représentent chacun, indépendamment les uns des autres, le chlore, un hydroxy, un méthoxy ou un méthyle.

5. Polymère chloré selon la revendication 1 dans lequel $R_1$ représente un phényle ou un radical de formule II dans lequel les radicaux $R_4$, $R_5$ et $R_6$ sont identiques.

6. Polymère chloré selon la revendication 1 dans lequel $R_1$ représente un phényle ou un radical de formule II dans lequel $n_1$ et $n_2$ sont égaux chacun à 0 et $n_3$ est égal a 3.

7. Polymère chloré selon la revendication 1 dans lequel $R_1$ représente un phényle.

8. Polymère chloré selon la revendication 1 dans lequel $R_2$ et $R_3$ représentent chacun un radical hydroxy-2 éthyle.

9. Polymère chloré selon la revendication 1 qui contient en plus au moins un carboxylate de Me(II) et/ou un phénolate ou alkyl phénolate de Me(II), le symbole Me(II) représentant Mg, Ca, Ba, Zn ou Cd et la partie carboxylate étant l'anion d'un acide carboxylique contenant de 7 à 20 atomes de carbone.

10. Polymère chloré selon la revendication 9 caractérisé en ce que le carboxylate de Me(II) est un mélange de carboxylate de baryum/zinc, de calcium/zinc ou de magnésium/zinc.

11. Polymère chloré selon la revendication 1 caractérisé en ce qu'il s'agit d'un poly-(chlorure de vinyle).

12. Composés répondant à la formule IA

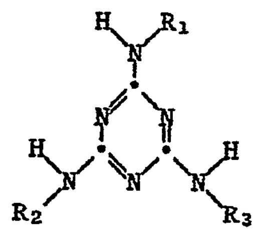

(IA)

dans laquelle $R_1$ représente un radical répondant à la formule II:

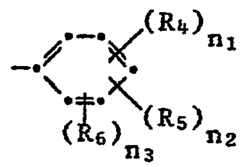

(II)

dans laquelle $R_4$, $R_5$ et $R_6$ représentent chacun, indépendamment les uns des autres, le chlore, le brome, un radical cyano, méthoxycarbonyle, éthyoxycarbonyle, carbamoyle, hydroxy, méthoxy ou éthoxy ou un radical alkyle en $C_1$–$C_{18}$, $n_1$, $n_2$ et $n_3$ représentent chacun un nombre égal à 0, à 1, à 2 ou à 3 avec la condition que la somme ($n_1+n_2+n_3$) soit égale à un nombre de 1 à 3, et $R_2$ et $R_3$ représentent chacun, indépendamment l'un de l'autre, un radical hydroxy-alkyle en $C_2$–$C_4$.

13. Composés selon la revendication 12, en l'espèce:

1a bis-(hydroxy-2 éthylamino)-2,4 (hydroxy-3 anilino)-6 triazine 1,3,5
1a bis-(hydroxy-2 éthylamino)-2,4 (chloro-2 anilino)-6 triazine 1,3,5
1a bis-(hydroxy-2 éthylamino)-2,4 (chloro-3 anilino)-6 triazine 1,3,5
1a bis-(hydroxy-2 éthylamino)-2,4 (chloro-4 anilino)-6 triazine 1,3,5, et
1a bis-(hydroxy-2 éthylamino)-2,4 (méthoxy-3 anilino)-6 triazine 1,3,5.

14. Application des composés de formule I qui ont été définis à la revendication 1 pour la stabilisation de polymères chlorés contre les effets destructeurs de la lumière et/ou de la chaleur.

## Claims

1. A chlorine-containing polymer containing at least one compound of the formula I

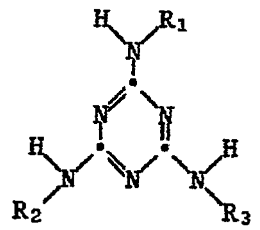

(I)

in which $R_1$ is phenyl or a group of the formula II

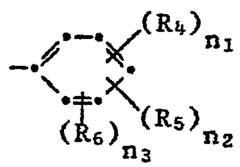

(II)

in which $R_4$, $R_5$ and $R_6$ independently of one another are chlorine, bromine, cyano, methoxycarbonyl, ethoxycarbonyl, carbamoyl, hydroxyl, methoxy, ethoxy or $C_1$–$C_{18}$-alkyl and $n_1$, $n_2$ and $n_3$ are 0, 1, 2 or 3, subject to the proviso that the sum of $n_1$+$n_2$+$n_3$ is an integer from 1 to 3, and $R_2$ and $R_3$ independently of one another are $C_2$–$C_4$-hydroxyalkyl.

2. A chlorine-containing polymer according to claim 1, in which $R_1$ is phenyl or a group of the formula II in which $R_4$, $R_5$ and $R_6$ independently of one another are chlorine, bromine, hydroxyl, methoxy, ethoxy or $C_1$–$C_{18}$-alkyl.

3. A chlorine-containing polymer according to claim 1, in which $R_1$ is phenyl or a group of the formula II in which $R_4$, $R_5$ and $R_6$ independently of one another are chlorine, hydroxyl, methoxy, ethoxy, or $C_1$–$C_{12}$-alkyl.

4. A chlorine-containing polymer according to claim 1, in which $R_1$ is phenyl or a group of the formula II in which $R_4$, $R_5$ and $R_6$ independently of one another are chlorine, hydroxyl, methoxy or methyl.

5. A chlorine-containing polymer according to claim 1, in which $R_1$ is phenyl or a group of the formula II in which the radicals $R_4$, $R_5$ and $R_6$ are identical.

6. A chlorine-containing polymer according to claim 1, in which $R_1$ is phenyl or a group of the formula II in which $n_1$ and $n_2$ are 0 and $n_3$ is 1.

7. A chlorine-containing polymer according to claim 1, in which $R_1$ is phenyl.

8. A chlorine-containing polymer according to claim 1, in which $R_2$ and $R_3$ are 2-hydroxyethyl.

9. A chlorine-containing polymer according to claim 1 containing, in addition, at least one Me(II) carboxylate and/or Me(II) phenate or alkylphenate in which Me(II) is Mg, Ca, Ba, Zn or Cd and carboxylate is the anion of a carboxylic acid having 7 to 20 C atoms.

10. A chlorine-containing polymer according to claim 9, wherein the Me(II) carboxylate is a mixture of a barium/zinc, calcium/zinc or magnesium/zinc carboxylate.

11. A chlorine-containing polymer according to claim 1, wherein the chlorine-containing polymer is polyvinyl chloride.

12. A compound of the formula IA

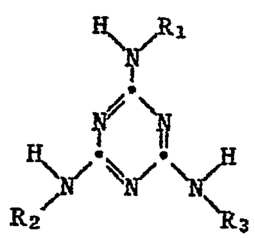

(IA)

in which $R_1$ is a group of the formula II

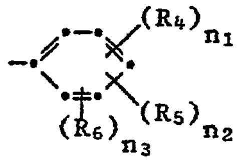

(II)

in which $R_4$, $R_5$ and $R_6$ independently of one another are chlorine, bromine, cyano, methoxycarbonyl, ethoxycarbonyl, carbamoyl, hydroxyl, methoxy, ethoxy or $C_1$–$C_{18}$-alkyl and $n_1$, $n_2$ and $n_3$ are 0, 1, 2 or 3, subject to the proviso that the sum of $n_1+n_2+n_3$ is an integer from 1 to 3, and $R_2$ and $R_3$ independently of one another are $C_2$–$C_4$-hydroxyalkyl.

13. The compounds
2,4-bis-(2-hydroxyethylamino)-6-(3-hydroxyanilino)-1,3,5-triazine,
2,4-bis-(2-hydroxyethylamino)-6-(2-chloroanilino)-1,3,5-triazine,
2,4-bis-(2-hydroxyethylamino)-6-(3-chloroanilino)-1,3,5-triazine,
2,4-bis-(2-hydroxyethylamino)-6-(4-chloroanilino)-1,3,5-triazine and
2,4-bis-(2-hydroxyethylamino)-6-(3-methoxyanilino)-1,3,5-triazine,
according to claim 12.

14. The use of the compounds of the formula I defined in claim 1 for stabilizing chlorine-containing polymers against the harmful effects of light and/or heat.